(19)

Europäisches Patentamt

European Patent Office

Office européen des brevets

(11) **EP 3 211 443 A1**

(12) **EUROPEAN PATENT APPLICATION**

(43) Date of publication:
**30.08.2017 Bulletin 2017/35**

(51) Int Cl.:
**G01R 33/56** *(2006.01)*        **G01R 33/567** *(2006.01)*
**A61B 5/055** *(2006.01)*

(21) Application number: **16157459.5**

(22) Date of filing: **25.02.2016**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicants:
• **Université d'Aix Marseille**
  **13284 Marseille Cedex 07 (FR)**
• **Centre National de la Recherche Scientifique**
  **75016 Paris (FR)**

(72) Inventors:
• **GIRARD, Olivier Maciej**
  **13005 Marseille (FR)**
• **DUHAMEL, Guillaume Didier David**
  **13011 Marseille (FR)**
• **CALLOT, Virginie**
  **13011 Marseille (FR)**

(74) Representative: **de Jong, Jean Jacques et al**
**Omnipat**
**24, place des Martyrs de la Résistance**
**13100 Aix en Provence (FR)**

(54) **METHOD FOR IMPROVING SPINAL CORD CONTRAST IN MAGNETIC RESONANCE IMAGING**

(57)    The invention relates to a method for producing an MRI image, comprising the steps of acquiring multiple intermediate images of a subject under analysis using alternating preparation phases; synchronizing the acquisitions with a physiological variable of the subject (ECG); forming a first set of intermediate images ({MTs}) and a second set of intermediate images ({MTd}) resulting from distinct preparation phases ; producing an output image (ihMT, ihMTR) based on a function of the intermediate images of the first and second sets; assigning to each intermediate image a timestamp (TRa) based on the time elapsed between the acquisition time of the current intermediate image and the acquisition time of the previous intermediate image; matching intermediate images of the first set with respective intermediate images of the second set based on their timestamps being equal within a tolerance margin ($\partial$TR); and using only the matched intermediate images ({MTs*}, {MTd*}) for producing the output image.

Fig 5

**EP 3 211 443 A1**

## Description

## Field

The invention relates to Magnetic Resonance Imaging (MRI) synchronized to a physiological variable, in particular to spinal cord imaging using inhomogeneous magnetization transfer (ihMT) techniques and synchronization to the subject's heart rate.

## Background

Magnetic resonance imaging may use multiple intermediate images separated by a repetition time TR for constructing a single end image that has correct contrast and signal-to-noise ratio. As many as 120 images may be required in some cases, separated by a repetition time of several seconds. Motion of a target organ during the intermediate image acquisitions causes blurring or ghosting. Although the subject may be perfectly still, some organs will inevitably move, such as the lungs and heart. The heart activity will even cause other organs to pulsate, such as the spinal cord. Indeed, the spinal cord bathes in a relatively large volume of cerebrospinal fluid that is affected by blood flow variations.

In order to overcome these difficulties, the acquisition of the intermediate images is usually synchronized to the particular physiological variable that causes the motion, so that each intermediate image is acquired at the same motion phase of the organ. In particular, for observing the spinal cord, the intermediate image acquisition is synchronized to the heart rate.

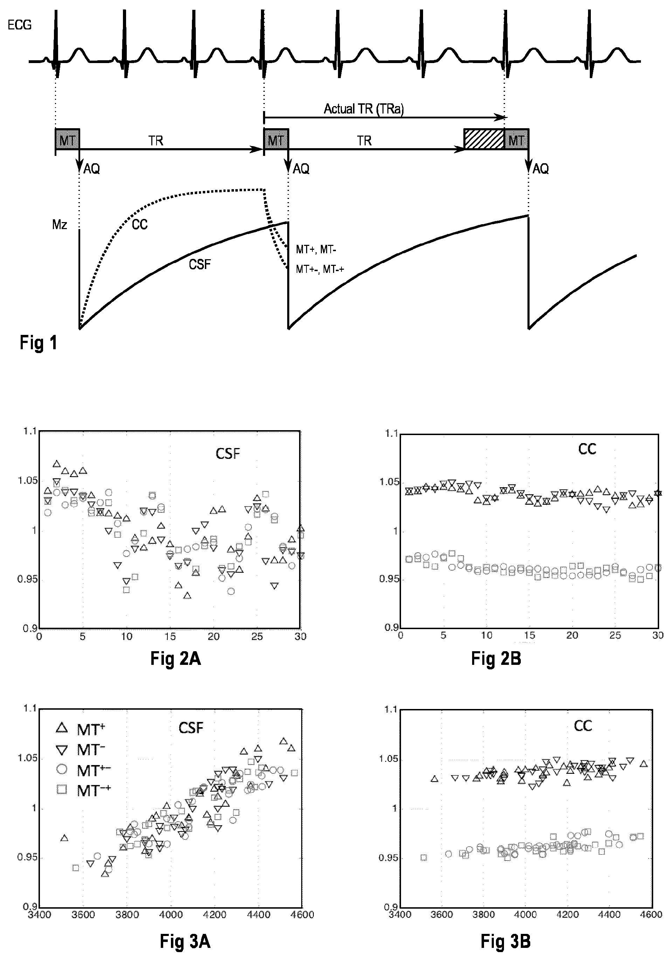

Figure 1 is a timing diagram that illustrates such synchronization, in the context of an inhomogeneous magnetization transfer technique (ihMT) that is particularly well suited for central nervous system imaging.

The top waveform illustrates an electrocardiogram ECG of the subject being observed. Synchronization is performed with the R-peaks of the ECG. A first R-peak starts a nominal repetition time interval TR. The actual image acquisition AQ starts after a preparation phase denoted MT, at a time when the heart motion or the spinal cord motion has subdued.

An image acquisition generally includes measuring the magnetization values of a voxel grid contained in a cross-section, or slice, being analyzed. Fast imaging strategies typically use the whole available longitudinal magnetization by tipping it into the transverse plane prior to the dedicated readout module. In that sense magnetization measurement is destructive, whereby a certain time is needed for the longitudinal magnetization to regenerate before the next measurement. The bottom waveforms of figure 1 illustrate, in bold lines, the magnetization regeneration for an essentially liquid tissue, such as cerebrospinal fluid CSF and, in dotted lines, the magnetization regeneration for a non-liquid tissue, such as corpus callosum CC. As shown approximately to scale, the magnetization regeneration is significantly slower for liquids (CSF). The repetition time TR is generally set based on the slowest magnetization regeneration time to allow full recovery.

In practice, the nominal repetition time TR is greater than the heart rate period. The repetition time may be in the order of 4.5 seconds, lasting for several heartbeats. The next acquisition is synchronized to the first R-peak following the end of the previous TR interval.

According to ihMT techniques, the preparation phases, or magnetization transfer (MT) phases, are used for modifying the properties of the magnetization depending on the tissue types. Four types of magnetization transfer phases are usually used in equal numbers. A first, denoted MT+, includes applying a burst of radiofrequency pulses having a single positive frequency offset with respect to the free-water Larmor frequency. The second, denoted MT-, includes applying a burst of radiofrequency pulses having a single negative frequency offset. The third, denoted MT+-, includes applying a burst of radiofrequency pulses that have a dual-frequency offset, i.e. pulses having offsets switched between positive and negative. The fourth, denoted MT-+, includes applying a burst of radiofrequency pulses that have a complementary dual-frequency offset, i.e. pulses having offsets switched between negative and positive.

In practice, each type of preparation phase is used in the order of 20 to 30 times, producing a total of 80 to 120 intermediate images, denoted MT-prepared images or simply MT images. Each quadruplet of MT images (MT+, MT-, MT+-, MT-+) may be used to produce an ihMT image in the form

$$ihMT_i = (MT_+)_i + (MT_-)_i - (MT_{+-})_i - (MT_{-+})_i.$$

The end ihMT image used for the MRI output is the sum or average of the images $ihMT_i$. Normalization may be applied to calculate the ihMT ratio (denoted ihMTR) by dividing the end ihMT image by a reference image denoted M0, acquired under similar conditions than the MT intermediate images, except that the amplitude of the RF burst is set to zero. The ihMTR image is often preferred for central nervous system analysis because it provides a quantitative metric (expressed in percentage).

If the target region is liquid, like CSF, the MT phases have no noticeable effect on the magnetization, as shown by the bold line regeneration waveform. If the target region is non-liquid, like CC, the MT phases cause an attenuation of the magnetization, with different degrees depending on the frequency offset sequence. Both the single-frequency offset MT phases (MT+, MT-) cause a substantially similar degree of attenuation. Both the dual-frequency offset MT phases (MT+-, MT-+) also cause a substantially similar degree of attenuation, but larger than that caused by the single-frequency offset MT phases (especially for myelinated tissues), as shown in the dotted line waveforms.

Thus ihMT or ihMTR images theoretically exhibit sharp contrast between liquid and non-liquid regions such as myelinated tissues, and are well suited for spinal cord examination. In some circumstances, however, contrast is not as sharp as expected.

## Summary

The present disclosure relates generally to a method for producing an MRI image, comprising the steps of acquiring multiple intermediate images of a subject under analysis using alternating preparation phases; synchronizing the acquisitions with a physiological variable of the subject; forming a first set of intermediate images and a second set of intermediate images resulting from distinct preparation phases; producing an output image based on a function of the intermediate images of the first and second sets; assigning to each intermediate image a timestamp based on the time elapsed between the acquisition time of the current intermediate image and the acquisition time of the previous intermediate image; matching intermediate images of the first set with respective intermediate images of the second set based on their timestamps being equal within a tolerance margin; and using only the matched intermediate images for producing the output image.

The function may be a difference between the intermediate images of the first and second sets.

The method may comprise the additional steps of preparing the acquisitions through magnetization transfer (MT) phases alternately using single-frequency offsets and dual-frequency offsets; including in the first set the intermediate images resulting from the single-frequency offset MT phases; and including in the second set the intermediate images resulting from the dual-frequency offset MT phases.

The method may comprise the additional steps of increasing the current tolerance margin by an increment; and reiterating the matching step to produce a current candidate output image.

The method may comprise the additional steps of identifying a reference area in the candidate images, producing an expected value in response to the preparation phases; discarding candidate images that produce values in the reference area that exceed an error margin about the expected value; and selecting for the output image the candidate image that produces the best signal-to-noise ratio among the remaining candidate images.

The reference area may correspond to tissue insensitive to the preparation phases.

The matching step may comprise cycling through the intermediate images by increasing timestamps or by decreasing timestamps.

The increment may be increased at each iteration following a geometrical progression.

The subject may include the spinal cord, the physiological variable may be the heart rate, and the reference area may be cerebrospinal fluid adjacent the cerebellum.

## Brief Description of Drawings

Other advantages and features will become more clearly apparent from the following description of particular embodiments of the invention provided for exemplary purposes only and represented in the appended drawings, in which:

- Figure 1, previously described, is a time diagram illustrating an ECG-synchronized image acquisition sequence in an MRI system;

- Figures 2A and 2B illustrate magnetization samples acquired in cerebrospinal fluid CSF and corpus callosum CC using four types of MT preparation phases, ordered by acquisition rank, in an ECG-synchronized MRI system;

- Figures 3A and 3B illustrate the samples of figures 2A and 2B, ordered by actual repetition time;

- Figure 4 is an image of a slice of a head showing a reference area;

- Figure 5 is a flowchart of a method for improving contrast in inhomogeneous MT images; and

- Figures 6A and 6B are normalized inhomogeneous MT images of a same slice of a head, before and after use of the contrast improving method.

**Description of Embodiments**

In figures 2A and 2B, 30 magnetization samples have been acquired experimentally for each of the four MT preparation phases. ECG synchronization was used in this case with a fixed nominal repetition time of 4.5 seconds. The samples of figure 2A pertain to cerebrospinal fluid CSF, while the samples of figure 2B pertain to corpus callosum CC. The samples are ordered by their acquisition rank 1-30. The upright triangles correspond to the MT+ prepared samples, the upside-down triangles to the MT-prepared samples, the squares to the MT-+ prepared samples, and the circles to the MT+- prepared samples.

The ihMT value resulting from figure 2A, i.e. the average of the differences between the single-frequency offset prepared samples (MT+, MT-) and the dual-frequency offset prepared samples (MT+-, MT-+), theoretically 0, has a relatively large standard deviation. The standard deviation in figure 2B is significantly smaller. This finding could be perceived as a reason behind the contrast degradation noted in ihMT images of CSF regions. Indeed, the poorer standard deviation of the samples of figure 2A is not only due to an unforeseeable factor, such as noise. It is mostly due to the variations of the actual repetition time.

As stated in relation with figure 1, showing ECG-synchronized acquisitions, each acquisition begins at the first R-peak of the ECG that follows the end of the previous nominal repetition interval TR. The repetition time is preferably short to reduce the acquisition time of the desired set of samples. However, the repetition time should allow sufficient recovery of the magnetization of the tissue of interest, so that the magnetization can be sampled with a satisfactory signal-to-noise ratio. As shown in figure 1, longitudinal magnetization recovers faster in corpus callosum CC than in cerebrospinal fluid CSF because of shorter longitudinal relaxation time ($T_1$). Typically, repetition times as low as 2.5 seconds are sufficient for CC to attain a stable state for an MRI system operating at 1.5 Tesla, whereas CSF would require a longer repetition time of about 10 seconds.

In fact, full recovery of the magnetization is not required for CSF, because the values are normally cancelled out in the resulting ihMT image leading to a zero ihMT signal. For this reason repetition times significantly lower than 10 seconds are used in practice, such as 2.5 or 4.5 seconds.

However, for the values to truly cancel out in the ihMT image, they should be sampled at the exact same moment of the magnetization recovery curve, which is difficult in practice. Indeed, the heart rate does not have a constant period. As shown in figure 1 as an example, the heart rate slows down in the second half of the diagram. As a consequence, the second acquisition AQ occurs with more delay after the end of a repetition interval TR than the first acquisition, additional delay during which the magnetization regrowth continues and departs from the value of the previous acquisition. The sampling position on the regrowth curve depends on the actual repetition time TRa, equal to the time interval between the current acquisition and the previous acquisition.

Even if the heart rate did not vary, it would be difficult to adjust the repetition time as an exact multiple of the heart rate period. In practice, the periods would inevitably be different, causing a beat phenomenon where the actual repetition time TRa varies between the nominal repetition time TR and TR + $T_{HR}$, where $T_{HR}$ is the average heart rate period.

Figures 3A and 3B show samples analogous to figures 2A and 2B, except that they are ordered by the actual repetition times TRa, expressed in milliseconds. The actual repetition times vary between 3.5 seconds and 4.6 seconds.

Figure 3A, corresponding to the CSF samples, shows a growth tendency similar to that of the CSF magnetization recovery between 3.5 and 4.6 seconds, having a relatively small standard deviation with respect to the recovery curve. In particular, there is no difference in trends between the four MT prepared data clouds, which is consistent with an absence of ihMT signal in this area (i.e. no signal difference between single- and dual-frequency offset MT phases)

Figure 3B, corresponding to the CC samples, is not significantly different than figure 2B, meaning that the actual repetition time has little influence on the absolute values of these samples, which is consistent with faster recovery as compared to CSF.

The growth tendency of figure 3A would not cause difficulties if the sample set contained quadruplets (MT+, MT-, MT+-, MT-+) that each have closely matched actual repetition times TRa. All the samples would then mutually cancel with sufficient accuracy when calculating the average of the differences (or the difference of the averages) for forming the end ihMT image.

In practice, however, since the heart rate or other physiological variables cannot be controlled, the set of samples will likely contain singularities that have actual repetition times that find no match. It is such singularities that cause contrast degradation in the resulting ihMT image of CSF areas, by causing a spurious non-zero ihMT signal. The likelihood of producing singularities increases when the nominal repetition time decreases, since the growth rate is steeper near the beginning of the recovery curve.

The inventors propose herein a method for compensating the influence of such singularities.

According to this method, a timestamp is assigned to each intermediate image, equal to the actual repetition time TRa, i.e. the time elapsed between the current image acquisition and the previous image acquisition. Data available in conventional MRI equipment may be used to produce such timestamps.

The single-frequency offset MT images are organized in a set denoted {MTs}, and the dual-frequency offset MT images are organized in a set denoted {MTd}. Here, "organizing images in sets" does not mean manipulating large volumes of image data, but manipulating image identifiers, such as the acquisition numbers and MT types.

Prior to achieving any operations with the images, a matching procedure is carried out, where the time stamps of the images in {MTs} are compared to the time stamps of the images in {MTd}. If a pair of compared time stamps are equal within a tolerance margin $\partial$TR, the corresponding images are marked as matched and may be removed from the remaining comparisons, i.e. avoiding multiple matches with a single image. The procedure continues with the remaining images until no more matches are found. Formally, it can be considered that each matching pair of images is moved from the sets {MTs} and {MTd} into two respective sets of matched images {MTs*} and {MTd*}, and that the procedure continues with the images remaining in the sets {MTs} and {MTd}.

At the end of the matching procedure, only the matched images, i.e. the sets {MTs*} and {MTd*}, are used for producing the end image. The difference and average calculations may be performed in any order with the images, since the operations are commutative.

The comparison procedure may be formalized generically by the following iterative inequality:

$$\text{TRa}_i - \partial\text{TR} \leq \text{TRa}_j \leq \text{TRa}_i + \partial\text{TR}$$

Where $\text{TRa}_i$ is the timestamp of an image in one of the sets {MTs}, {MTd}, with index *i* cycling through the images in the set, and $\text{TRa}_j$ is the timestamp of an image in the other of the sets {MTd}, {MTs}, with index *j* cycling through the images in the other set. The indexes *i* and *j* may cycle through the images in an arbitrary order, for instance by order of acquisition, such as the order shown in figures 2A and 2B. In that case, as matching pairs are found and removed from the pool, i.e. sets {MTs} and {MTd}, points disappear in arbitrary locations in the clouds of figures 2A and 2B, assuming these clouds represent the pool.

The above simple comparison criterion does not necessarily find the "closest" matches for each pair if the indexes are cycled arbitrarily or by order of acquisition, which may have side effects, such as the exclusion of possible matches.

To reduce the number of exclusions, the indexes may be cycled by order of increasing timestamps, such as the order shown in figures 3A and 3B. In that case, as matching pairs are found and removed from the pool, points disappear from left to right in the clouds of figures 3A and 3B, and matches are found almost immediately, since the comparisons start among the images having the closest timestamps. A similar result is obtained by cycling through the indexes by order of decreasing timestamps.

For this purpose, the images from both sets may be organized in a single-dimensional array indexed by increasing time stamps (or decreasing time stamps). The matching procedure then starts at the first record of the array, identifying a first image that may belong to any one of the sets {MTs} and {MTd}, and steps through the array up to a record identifying an image belonging to the other set, or complementary set. This second image necessarily has a timestamp closest to that of the first image among the images of the complementary set. The time stamps are compared and the two records are removed from the array if the tolerance margin is satisfied. If the tolerance margin is not satisfied, this means that the first image has no match, and the records are maintained in the array. It is not necessary to step through the whole array to find a matching image; the process may stop as soon as the currently examined record contains a time stamp that is beyond the tolerance margin with respect to the starting record. In any case, the procedure is reiterated starting from the second record of the array, and so forth until the array is reduced to the images that find no match.

The resulting matching pairs may be odd in number, meaning that the sets {MTs*} and {MTd*} may not contain together an integer number of quadruplets (MT+, MT-, MT+-, MT-+). In practice, this will have no consequence on CSF regions of the end image, and only negligible consequences on the CC regions, bearing in mind that the discarded images could have consequences that are more deleterious on regions containing CSF if they were used.

If the tolerance margin $\partial$TR is too low, there may be insufficient matching intermediate images to offer a satisfactory signal-to-noise ratio in the end image. The matching procedure may be reiterated several times, increasing the tolerance margin $\partial$TR by an increment $\partial$t each time. Multiple candidate images $\text{ihMT}_i$ are thus produced using an increasing number of images from the sets {MTs} and {MTd} or the array. The procedure is ended when a stop criterion is reached, such as a threshold value for the margin $\partial$TR.

In the example of an ECG synchronization, yielding samples that have timestamps that may differ by up to approximately 1.5 seconds, the initial value of $\partial$TR may be 2.5 milliseconds and the increment $\partial$t may be 2.5 milliseconds. The increment $\partial$t may increase geometrically at each iteration, for instance taking values 2.5, 5, 7.5, 10, 15, 20 milliseconds...

An alternative stop criterion may be a threshold signal-to-noise ratio in the form of a standard deviation produced when calculating the averages in the candidate images. However, such a criterion does not guarantee the accuracy of the end image, in particular in the CSF regions.

To guarantee a target accuracy, a reference area may be used in the imaging slice under analysis, corresponding to a CSF region that is known to have a zero ihMT value.

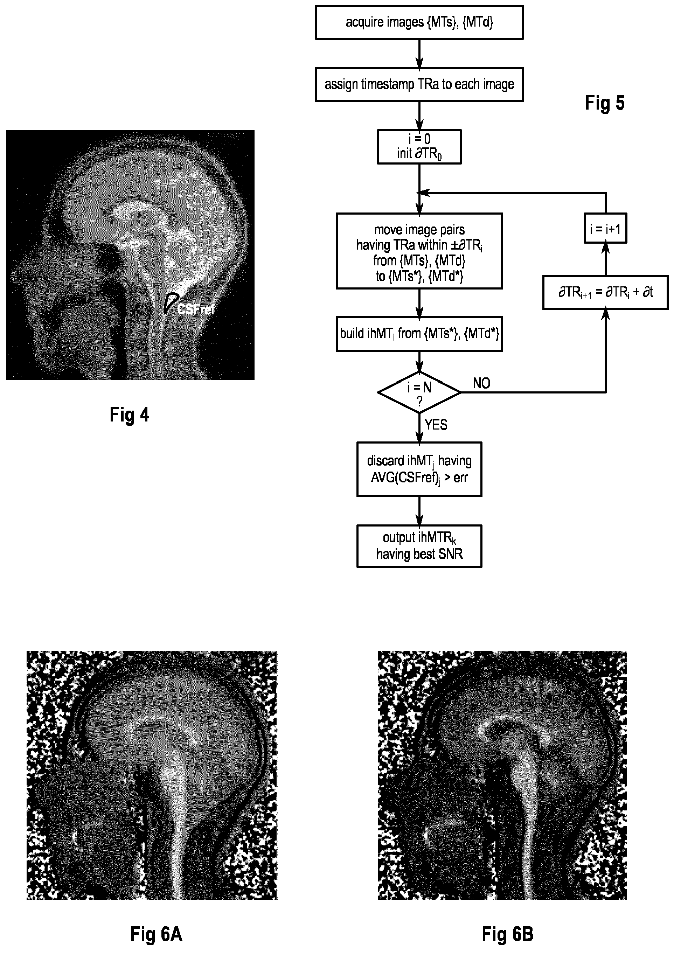

As shown in figure 4, a reference area CSFref may be defined on the first produced image (such as M0 or $MT_+$), that is accurate enough to distinguish the CSF regions. The reference area may be defined manually by the user, using a pointer to draw on a screen, or identified automatically by image recognition techniques.

A satisfactory choice for the reference area may be the CSF adjacent the cerebellum, as shown, area which is easy to identify and immune to heart pulsations.

With the so identified reference area, where the values are normally 0 in the end ihMT image, the compensation method may be completed as follows. After each iteration i of the tolerance margin $\partial$TR, the resulting image sets {MTs*} and {MTd*} are used to produce a candidate end image $ihMT_i$ as shown in figure 4.

After all iterations, where *i* has been incremented from 0 to N, the average value in the reference area CSFref for each candidate image $ihMT_j$ is calculated and compared to an error threshold err. The candidate images producing an average above the error threshold are discarded. A satisfactory error threshold may be 0.3 % of the maximum signal level (i.e. in terms of ihMTR).

The remaining candidate ihMT images all produce an average in the CSFref area below the error threshold, especially the first ones, that were produced with the smaller values of the tolerance margin $\partial$TR. However, the first candidate images are produced from fewer MT images, and are likely to have a poor SNR. The final candidate image used for the output may be one that has the best SNR among the remaining candidate images.

Figure 5 is an exemplary flowchart summarizing the above contrast improving technique. It should be self-explanatory in conjunction with the terminology and abbreviations used above.

Figures 6A and 6B are ihMTR images produced respectively without and with an exemplary implementation of the above described contrast improving techniques. In figure 6B, the CSF regions are practically black (i.e. have 0 signal) whereas they are grey in figure 6A. As a consequence, the spinal cord, which is a shade of grey, stands out much better in figure 6B.

Although embodiments of the invention were described in the context of MRI techniques adapted to the central nervous system, using MT-preparation phases and a reference area CSFref corresponding to cerebrospinal fluid, the invention may apply to other MRI techniques using different preparation phases and reference areas. Preparation phases are generally intended to introduce magnetic signatures that differ according to the explored tissues, allowing discrimination of tissue that is insensitive to the preparation phases (e.g. CSF), producing zero difference values, from tissue that is sensitive (e.g. CC), producing non-zero difference values. Generally, the reference area would thus be chosen in tissue insensitive to the preparation phases.

In other applications, the reference area may be chosen in a region that has a known response to the preparation phases, producing a predictable non-zero image value. The reference area may be chosen on a template object placed within the area under analysis. The error threshold mentioned above is then an error margin about the expected non-zero value.

**Claims**

1. Method for producing an MRI image, comprising the following steps:

   - acquiring multiple intermediate images of a subject under analysis using alternating preparation phases;
   - synchronizing the acquisitions with a physiological variable of the subject (ECG);
   - forming a first set of intermediate images ({MTs}) and a second set of intermediate images ({MTs}) resulting from distinct preparation phases ;
   - producing an output image (ihMT, ihMTR) based on a function of the intermediate images of the first and second sets;

   **characterized in that** it comprises the following steps:

   - assigning to each intermediate image a timestamp (TRa) based on the time elapsed between the acquisition time of the current intermediate image and the acquisition time of the previous intermediate image;
   - matching intermediate images of the first set with respective intermediate images of the second set based on their timestamps being equal within a tolerance margin ($\partial$TR); and

• using only the matched intermediate images ({MTs*}, {MTd*}) for producing the output image.

2. The method of claim 1, wherein the function is a difference between the intermediate images of the first and second sets.

3. The method of claim 1, comprising the following additional steps:

• preparing the acquisitions through magnetization transfer (MT) phases alternately using single-frequency offsets (MT+, MT-) and dual-frequency offsets (MT+-, MT-+);
• including in the first set the intermediate images ({MTs}) resulting from the single-frequency offset MT phases; and
• including in the second set the intermediate images ({MTd}) resulting from the dual-frequency offset MT phases.

4. The method of claim 1, comprising the following additional steps:

• increasing the current tolerance margin ($\partial TR_i$) by an increment ($\partial t$); and
• reiterating the matching step to produce a current candidate output image ($ihMT_i$).

5. The method of claim 4, comprising the following additional steps:

• identifying a reference area (CSFref) in the candidate images, producing an expected value in response to the preparation phases;
• discarding candidate images that produce values in the reference area that exceed an error margin (err) about the expected value; and
• selecting for the output image the candidate image that produces the best signal-to-noise ratio among the remaining candidate images.

6. The method of claim 5, wherein the reference area corresponds to tissue insensitive to the preparation phases.

7. The method of claim 4, wherein the matching step comprises cycling through the intermediate images by increasing timestamps or by decreasing timestamps.

8. The method of claim 4, wherein the increment ($\partial t$) is increased at each iteration following a geometrical progression.

9. The method of claim 6, wherein the subject includes the spinal cord, the physiological variable is the heart rate, and the reference area is cerebrospinal fluid adjacent the cerebellum.

**Fig 1**

**Fig 2A**

**Fig 2B**

**Fig 3A**

**Fig 3B**

**Fig 5**

**Fig 4**

**Fig 6A**

**Fig 6B**

Europäisches
Patentamt

European
Patent Office

Office européen
des brevets

# EUROPEAN SEARCH REPORT

Application Number

EP 16 15 7459

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | OLIVIER M. GIRARD ET AL: "Magnetization transfer from inhomogeneously broadened lines (ihMT): Improved SC imaging using ECG synchronization", PROCEEDINGS OF THE INTERNATIONAL SOCIETY FOR MAGNETIC RESONANCE IN MEDICINE, 22ND ANNUAL MEETING AND EXHIBITION, MILAN, ITALY, 10-16 MAY 2014, vol. 22, 25 April 2014 (2014-04-25), page 3450, XP040664519, * the whole document * | 1-9 | INV. G01R33/56 G01R33/567 ADD. A61B5/055 |
| T | OLIVIER M. GIRARD ET AL: "Magnetization transfer from inhomogeneously broadened lines (ihMT): Improved imaging strategy for spinal cord applications", MAGNETIC RESONANCE IN MEDICINE., 9 March 2016 (2016-03-09), pages n/a-n/a, XP055292130, US ISSN: 0740-3194, DOI: 10.1002/mrm.26134 * the whole document * | 1-9 | |
| A | VARMA G ET AL: "Interpretation of magnetization transfer from inhomogeneously broadened lines (ihMT) in tissues as a dipolar order effect within motion restricted molecules", JOURNAL OF MAGNETIC RESONANCE, vol. 260, 7 September 2015 (2015-09-07), pages 67-76, XP029321534, ISSN: 1090-7807, DOI: 10.1016/J.JMR.2015.08.024 * the whole document * | 1-9 | TECHNICAL FIELDS SEARCHED (IPC) G01R A61B |

-/--

The present search report has been drawn up for all claims

| Place of search | Date of completion of the search | Examiner |
|---|---|---|
| Munich | 5 August 2016 | Raguin, Guy |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 16 15 7459

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | OLIVIER M. GIRARD ET AL: "Magnetization transfer from inhomogeneously broadened lines (ihMT): Qualitative evaluation of ihMT specificity toward myelinated structures", PROCEEDINGS OF THE INTERNATIONAL SOCIETY FOR MAGNETIC RESONANCE IN MEDICINE, 22ND ANNUAL MEETING AND EXHIBITION, MILAN, ITALY, 10-16 MAY 2014, vol. 22, 25 April 2014 (2014-04-25), page 1498, XP040662567, * the whole document * | 1-9 | |
| A | US 2016/041246 A1 (ALSOP DAVID [US] ET AL) 11 February 2016 (2016-02-11) * the whole document * | 1-9 | |
| A | US 2014/046168 A1 (KASSAI YOSHIMORI [JP] ET AL) 13 February 2014 (2014-02-13) * the whole document * | 1-9 | TECHNICAL FIELDS SEARCHED (IPC) |

The present search report has been drawn up for all claims

| Place of search | Date of completion of the search | Examiner |
|---|---|---|
| Munich | 5 August 2016 | Raguin, Guy |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 16 15 7459

This annex lists the patent family members relating to the patent documents cited in the above-mentioned European search report.
The members are as contained in the European Patent Office EDP file on
The European Patent Office is in no way liable for these particulars which are merely given for the purpose of information.

05-08-2016

| Patent document cited in search report | Publication date | Patent family member(s) | Publication date |
|---|---|---|---|
| US 2016041246 A1 | 11-02-2016 | EP 2967425 A2<br>US 2016041246 A1<br>WO 2014151596 A2 | 20-01-2016<br>11-02-2016<br>25-09-2014 |
| US 2014046168 A1 | 13-02-2014 | US 2004059213 A1<br>US 2007265522 A1<br>US 2012041299 A1<br>US 2014046168 A1 | 25-03-2004<br>15-11-2007<br>16-02-2012<br>13-02-2014 |

EPO FORM P0459

For more details about this annex : see Official Journal of the European Patent Office, No. 12/82